# EUROPÄISCHE PATENTSCHRIFT

(11) **EP 1 680 037 B1**
(45) Veröffentlichungstag und Bekanntmachung des Hinweises auf die Patenterteilung: **04.04.2007**
(21) Anmeldenummer: 04790816.5
(22) Anmeldetag: 25.10.2004
(51) Int. Cl.: A61B 18/14, A61B 17/28, A61B 17/22

(54) **MEDIZINISCHES INSTRUMENT MIT ENDOSKOP**
MEDICAL INSTRUMENT COMPRISING AN ENDOSCOPE
INSTRUMENT MEDICAL MUNI D'UN ENDOSCOPE

(30) Priorität: 24.10.2003 DE 10349825
(43) Veröffentlichungstag der Anmeldung: 19.07.2006
(73) Patentinhaber: Karl Storz GmbH & Co. KG, 78532 Tuttlingen (DE)
(72) Erfinder: BACHER, Uwe, 78532 Tuttlingen (DE); BARKI, Gérard, CH-1206 Genf (CH)
(74) Vertreter: Heuckeroth, Volker
(86) Internationale Anmeldenummer: PCT/EP2004/012028
(87) Internationale Veröffentlichungsnummer: WO 2005/039427

(56) Entgegenhaltungen:
- WO-A-02/094341
- US-A- 5 226 908
- US-A- 5 827 323
- US-B1- 6 419 626

## Beschreibung

Die vorliegende Erfindung betrifft ein medizinisches Instrument, mit einem Schaft, einem Arbeitselement am distalen Ende des Schaftes, einer Handhabe mit zumindest einem beweglichen Teil zum Betätigen des Arbeitselementes und mit einem Befestigungselement zum Befestigen eines Endoskops, das einen Endoskopschaft und ein Okular aufweist, wobei das distale Ende des Endoskops im Bereich des distalen Endes des Schaftes und das proximale Ende des Endoskops im Bereich der Handhabe des Instruments anbringbar sind und das Endoskop in Längsrichtung des Schafts verschiebbar ist.

Medizinische Instrumente gemäß dem Oberbegriff des Anspruchs 1, an denen ein Endoskop verschiebbar angebracht ist, sind z.B. aus den Dokumenten DE 40 42 102 C2 oder EP 0 117 894 A2 bekannt.

Mit der starken Verbreitung der minimal-invasiven Chirurgie wurde eine große Anzahl medizinischer Instrumente hierfür entwickelt. Bei der minimal-invasiven Chirurgie werden Operationen nur durch einen sehr kleinen Einschnitt innerhalb des menschlichen Körpers durchgeführt.

Die Beobachtung einer minimal-invasiv durchgeführten Operation erfolgt hierbei über eines oder mehrere Endoskope, die über die selbe oder eine weitere Öffnung an die Operationsstelle innerhalb des Körpers herangeführt werden.

Die Verwendung eines medizinischen Instruments und eines davon unabhängigen Endoskops hat allerdings den Nachteil, dass sich die relative Position des Endoskops zum Instrument beim Bewegen eines der beiden ändert, und damit auch die Perspektive, die sich dem Operateur bietet. Dies ist vor allem bei langwierigen Operationen für den Operateur ermüdend.

Da die Beobachtung einer Operation außerdem im Allgemeinen nicht als Stereobild erfolgt, ist die relative Lage des Endoskops zum Instrument und des Instruments zur Operationsstelle oft nur schwer zu beurteilen.

Außerdem erschwert die Verwendung eines medizinischen Instruments und eines davon unabhängigen Endoskops die Durchführung von Operationen durch nur eine Person, da normalerweise zumindest ein Operationshelfer zum Nachführen des Endoskops benötigt wird.

Aus diesem Grund wurden medizinische Systeme entwickelt, die aus einem medizinischen Instrument bestehen, an das ein Endoskop gekoppelt ist.

Ein solches System ist z.B. im Dokument US 4,759,348 beschrieben. In dem in diesem Dokument beschriebenen System wird das distale Ende eines flexiblen Endoskops am distalen Ende eines medizinischen Instruments befestigt. Das System zeichnet sich durch eine hohe mechanische Einfachheit aus. Außerdem kann das Endoskop so gestaltet sein, dass es an einem beliebigen medizinischen Instrument angebracht werden kann.

Das Endoskop ist allerdings nur am distalen Ende des Schaftes befestigt, was zu einer deutlichen Erhöhung des Instrumentendurchmessers führt. Dies erschwert das Einführen des medizinischen Systems in den Körper deutlich.

Des Weiteren wird dieses bekannte Endoskop bei einer Bewegung der beweglichen Teile des medizinischen Instruments nicht automatisch nachgeführt. Verfügt das medizinische Instrument also über ein Arbeitselement, das entlang der Längsachse relativ zum Instrumentenschaft verschiebbar ist, wird das Endoskop nicht mitverschoben. Das bedeutet, dass sich die Perspektive des Arbeitselements, die sich dem Operateur ergibt, beim Verschieben desselben ändert. Dies wiederum erschwert den präzisen Einsatz des Arbeitselements.

Das Dokument US 5,667,472 beschreibt ein ähnliches System, wobei optische Fasern entlang des gesamten Schaftes des medizinischen Instruments geführt werden. Hierdurch wird der Instrumentendurchmesser kaum merklich erhöht und sowohl das Einführen wie auch das Manövrieren des Instruments während der Operation werden deutlich erleichtert.

Allerdings fehlt bei diesem medizinischen System ebenfalls eine Nachführung des Endoskops mit dem Arbeitselement des medizinischen Instruments, was zu den oben beschriebenen Problemen führt.

Das Dokument DE 40 42 102 C2 beschreibt ein medizinisches Instrument, in das ein Endoskop integriert ist. Das Endoskop steht hierbei in Wirkverbindung mit dem Arbeitselement des medizinischen Instruments und wird bei Verschieben des Arbeitselements mitverschoben. Der Schaft des Endoskops liegt hierbei im Inneren des Schafts des Instruments. Das Okular des Endoskops ist am proximalen Ende des Instruments angeordnet, so dass die Längsachse des Okulars mit der Schaftachse des Instruments zusammenfällt.

Dieses System hat allerdings den Nachteil, dass das Endoskop ein integraler Bestandteil des medizinischen Instrumentes ist. So muss für jedes neue medizinische Instrument gleichzeitig ein Endoskop erworben werden.

Das Integrieren des Endoskops in das medizinische Instrument erschwert außerdem das Zerlegen zum Reinigen und Sterilisieren.

Weiterhin erweist sich diese Konstruktion für viele HFchirurgische Instrumente als ungeeignet.

Da bei HF-Instrumenten das Arbeitselement und die zugehörigen Leiter für die HF-Spannung regelmässig zum Austausch oder zur Sterilisation entfernt werden müssen, sind diese im Allgemeinen als gerade Drähte mit Werkzeugen am distalen Ende ausgebildet. Die Drähte können von dem distalen Ende in den Schaft eingeführt werden und werden am proximalen Ende mit einem Klemmkontakt befestigt. An dem proximalen Ende befindet sich auch der Anschluss für die externe Zuleitung der HF-Spannung. Somit ist bei solchen HF-Instrumenten am proximalen Ende der Raum zum Anbringen einer Endoskopoptik stark eingeschränkt.

Das Dokument EP 0 117 894 A2 beschreibt ein Instrument ähnlich dem in DE 40 42 102 C2 beschriebenen. Der Hauptunterschied besteht darin, dass das Endoskop innerhalb eines medizinischen Greifinstrumentes geführt wird.

Aus dem Dokument US-B1-6 419 626 ist ein chirurgisches endoskopisches Instrument bekannt, das einen Schaft, ein Arbeitselement am distalen Ende des Schaftes, eine Handhabe mit zumindest einem beweglichen Teil zum Betätigen des Arbeitselements und ein Befestigungselement zum Befestigen eines Endoskops aufweist, das einen Endoskopschaft aufweist, wobei das distale Ende des Endoskops im Bereich des distalen Endes des Schaftes und das proximale Ende des Endoskops im Bereich der Handhabe des Instruments anbringbar sind und das Endoskop in Längsrichtung des Schafts verschiebbar ist. Am proximalen Ende des Endoskops ist ein Bildschirm angeordnet, auf dem das endoskopische Bild dargestellt wird. Das Endoskop verläuft im Inneren des Instrumentenschafts.

Aus dem Dokument US 5 827 323 A ist ein medizinisches Instrument bekannt, durch dessen Schaft ein flexibles Endoskop hindurchgeschoben werden kann, so dass das distale lichteintrittsseitige Ende des Endoskops an einer Stelle proximal von einem Arbeitselement des Instruments aus dem Schaft heraustritt, so dass das Arbeitselement im Blickfeld des Endoskops liegt.

US 5 226 908 A offenbart ein chirurgisches Instrument, an das ein Endoskop ankoppelbar ist. Das Okular des Endoskops weist einen sich senkrecht zur Längsachse des Schafts des Instruments erstreckenden und verschwenkbar mit dem Schaft des Instruments verbundenen Verbindungsabschnitt auf, von dem sich der Hauptabschnitt des Okulars im rechten Winkel weg nach proximal erstreckt.

Das Dokument WO 02/094341 A offenbart ein chirurgisches System mit einem Endoskop, und einem Instrument mit Werkzeugen am distalen Ende, wobei die Werkzeuge an das distale Ende des Endoskops ankoppelbar sind.

Es ist eine Aufgabe der Erfindung, ein medizinisches Instrument anzugeben, das mit einem beliebigen Endoskop verschiebbar gekoppelt werden kann.

Hinsichtlich des oben genannten medizinischen Instruments wird die Aufgabe dadurch gelöst, dass das Instrument ein Befestigungselement aufweist, das so ausgebildet ist, dass das Endoskop an der Außenseite des Instruments anbringbar ist, wobei das Okular des Endoskops im am Instrument angebrachten Zustand außerhalb der Längsachse des Schaftes des Instruments liegt.

Durch das Befestigungselement an der Außenseite des Instruments kann ein erfindungsgemäßes Instrument mit einem beliebigen Endoskop verbunden werden. Damit kann z.B. ein einzelnes Endoskop mit einer Vielzahl medizinischer Instrumente gekoppelt werden, was zu einer deutlichen Kostenersparnis führt. Außerdem kann ein bereits vorhandenes Endoskop mit einem beliebigen neuen Instrument gemäß der Erfindung verbunden werden.

Weiterhin reicht es hierbei, bei Verschleiß des Instruments nur dieses zu ersetzen.

Dadurch, dass das Befestigungselement so ausgestaltet ist, dass das Okular des Endoskops außerhalb der Längsachse des Schaftes des Instruments liegt, kann das Endoskop an die Form des medizinischen Instruments angepasst werden. Somit kann insbesondere ein HF-Instrument, das an seinem proximalen Ende einen Anschluss für eine externe HF-Leitung aufweist, mit einem Endoskop ausgerüstet werden.

Die Verschiebbarkeit des Endoskops relativ zum medizinischen Instrument bleibt hierbei jedoch vollkommen erhalten.

In einer bevorzugten Ausführungsform weist das Arbeitselement zumindest eine mit einer HF-Spannung beaufschlagbare Elektrode auf, wobei das Arbeitselement vorzugsweise als bipolares Arbeitselement und weiter vorzugsweise als bipolare Zange ausgebildet ist.

In der modernen minimal-invasiven Chirurgie werden häufig Instrumente verwendet, deren Arbeitselemente als Elektrode oder Elektroden ausgebildet sind, die mit HF-Spannung beaufschlagt werden. Solche Instrumente werden häufig als bipolare Instrumente, z.B. als bipolare Zangen, ausgebildet.

Diese Instrumente können z.B. dazu verwendet werden, Gewebe zu schneiden und gleichzeitig Blutungen an der Operationsstelle zu stillen.

In einer weiteren bevorzugten Ausführungsform steht das Arbeitselement mit einem elektrischen Kontakt am proximalen Ende des Instruments in Verbindung, wobei der elektrische Kontakt auf der Längsachse des Schafts liegt.

Da die mit HF-Spannung beaufschlagbaren Arbeitselemente und die mit ihnen in Verbindung stehenden Leiter für die HF-Spannung auf Grund von Abnutzung oder zur Sterilisation häufig aus dem medizinischen Instrument ausgebaut werden müssen, hat es sich als zweckmäßig erwiesen, die Leiter für die HF-Spannung als gerade Drähte auszubilden, an deren distalem Ende sich die Arbeitselemente befinden. Dadurch können die Leiter und die damit verbundenen Arbeitselemente einfach am distalen Ende des Schafts in den Schaft eingeführt und dann am proximalen Ende des Instruments befestigt werden.

Dabei hat es sich als am zweckmäßigsten erwiesen, die Kontakte zur Zuleitung der externen HF-Spannung axial in der Längsachse des Schafts anzuordnen. Durch die vorliegende Erfindung wird nun gerade die Kombination eines solchen HF-Instruments mit einem Endoskop ermöglicht, da das Okular des daran anzubringenden Endoskops außerhalb der Längsachse des Instrumentenschafts liegt, so dass der HF-Anschluss auf der Längsachse des Schafts angeordnet sein kann.

In einer Ausgestaltung der zuvor genannten Maßnahme ist das Arbeitselement durch zumindest einen Leiter mit dem elektrischen Kontakt verbunden, wobei das Arbeitselement, der zumindest eine Leiter und der elektrische Kontakt als eine Einheit aus dem Schaft entnehmbar sind.

Diese Maßnahme erleichtert den Auseinander- und Zusammenbau des medizinischen Instruments deutlich, da die oft dünnen Leiter nicht mehr in dem Instrument in den elektrischen Kontakt eingeführt werden müssen.

Des Weiteren ermöglicht es diese Maßnahme, die stromführenden Bauteile des Instruments separat zusammenzubauen und zu testen und dann als Einheit in das Instrument einzusetzen. Dadurch wird es einfacher, die Funktionsfähigkeit der stromführenden Bauteile des Instruments sicherzustellen.

In einer weiteren bevorzugten Ausführungsform ist der elektrische Kontakt als Steckverbindung zum Anschluss eines externen Kabels ausgebildet, wobei die Steckverbindung im Wesentlichen in gradliniger Verlängerung des Schafts angeordnet ist.

Das Anbringen der Steckverbindung in einer im Wesentlichen gradlinigen Verlängerung des Schafts führt zu einer deutlich geringeren Beeinträchtigung der Bewegungsfreiheit eines Operateurs im Vergleich zu Zuleitungen, die seitlich am Instrument abstehen.

In einer bevorzugten Ausführungsform der Erfindung ist am Schaft des Instruments ein Führungsrohr angeordnet, das sich parallel zum Schaft erstreckt und in das der Endoskopschaft einführbar ist.

Durch ein solches Führungsrohr wird vermieden, dass ein Verschieben des Endoskops umliegendes Gewebe einklemmt und schädigt.

In einer weiteren bevorzugten Ausführungsform wird das Führungsrohr zum Aufnehmen des Endoskops durch einen Kanal im Schaft gebildet.

Diese Maßnahme hat den Vorteil, dass dadurch der Instrumentendurchmesser nur geringfügig vergrößert wird. Außerdem weist diese Ausführungsform nur wenige Ecken und Nischen auf, in denen sich Bakterien ansiedeln können und die schwer zu reinigen sind.

In einer weiteren bevorzugten Ausführungsform weist das Instrument einen Spülanschluss auf, derart dass eine Innenseite des Schafts und des Führungsrohrs gleichzeitig gespült werden kann.

Diese Maßnahme hat den Vorteil, dass auf konstruktiv einfache Weise sowohl die Arbeitselemente als auch die Optik eines anzubringenden Endoskops und die Umgebung der Operationsstelle gespült werden können.

In einer bevorzugten Ausführungsform steht das Befestigungselement mit dem beweglichen Teil der Handhabe in Wirkverbindung.

Diese Maßnahme hat den Vorteil, dass das Verschieben des Endoskops und des Arbeitselements mit einer Hand und in einer einzelnen Bewegung erfolgen kann. Hierdurch kann des Weiteren ein ungewolltes Verschieben des Endoskops gegenüber dem Schaft des Instruments vermieden werden. Somit wird die Ermüdung beim Einsatz des medizinischen Instruments mit dem Endoskop im Zuge von Operationen deutlich vermindert.

In einer weiteren bevorzugten Ausführungsform ist das Befestigungselement so ausgebildet, dass bei Betätigen der Handhabe das distale Ende des Endoskops in konstantem Abstand zum distalen Ende des Arbeitselements bleibt.

Diese Maßnahme ist besonders vorteilhaft, da das Endoskop dem Arbeitselement automatisch nachgeführt wird und dadurch der Operateur stets die gleiche Perspektive des Arbeitselements hat. Dies erhöht die Präzision von Operationen, die mit einem erfindungsgemäßen medizinischen System durchgeführt werden deutlich. Außerdem sind keine weiteren Maßnahmen zum Positionieren und zum Nachführen des Endoskops notwendig.

In einer weiteren bevorzugten Ausführungsform weist die Handhabe eines erfindungsgemäßen medizinischen Instruments zumindest einen Griff auf, der seitlich vom Instrument absteht.

Ein seitlich abstehender Griff erleichtert das präzise Verdrehen des medizinischen Instruments um seine Längsachse während einer Operation.

Außerdem kann durch einen seitlich abstehenden Griff der Winkel zwischen Griff und Okular des Endoskops deutlich vergrößert werden, was sowohl die Benutzung des Griffs als auch des Okulars des Endoskops während einer Operation deutlich vereinfacht.

In einer weiteren bevorzugten Ausführungsform der oben genannten Maßnahme ist der Griff der Handhabe in der Art von Scherengriffteilen ausgebildet.

Scherengriffteile haben sich für die einhändige Benutzung eines medizinischen Instruments gemäß der Erfindung als besonders ergonomisch vorteilhaft erwiesen.

In einer weiteren bevorzugten Ausführungsform ist das Befestigungselement so ausgebildet, dass ein Schaft des Endoskops in einem proximalen Abschnitt eine Krümmung aufweist und zumindest in dem gekrümmten Bereich flexibel oder biegsam ist.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese nachfolgend beschrieben:
- Fig. 1: eine Seitenansicht eines medizinischen Instruments mit einem daran angebrachten Endoskop,
- Fig. 2: eine vergrößerte Ansicht des distalen Bereichs des medizinischen Instruments aus Fig. 1,
- Fig. 3: ein Seitenansicht eines weiteren medizinischen Instruments, und
- Fig. 4: das medizinische Instrument aus Fig. 3, an das ein Endoskop angebracht wurde.

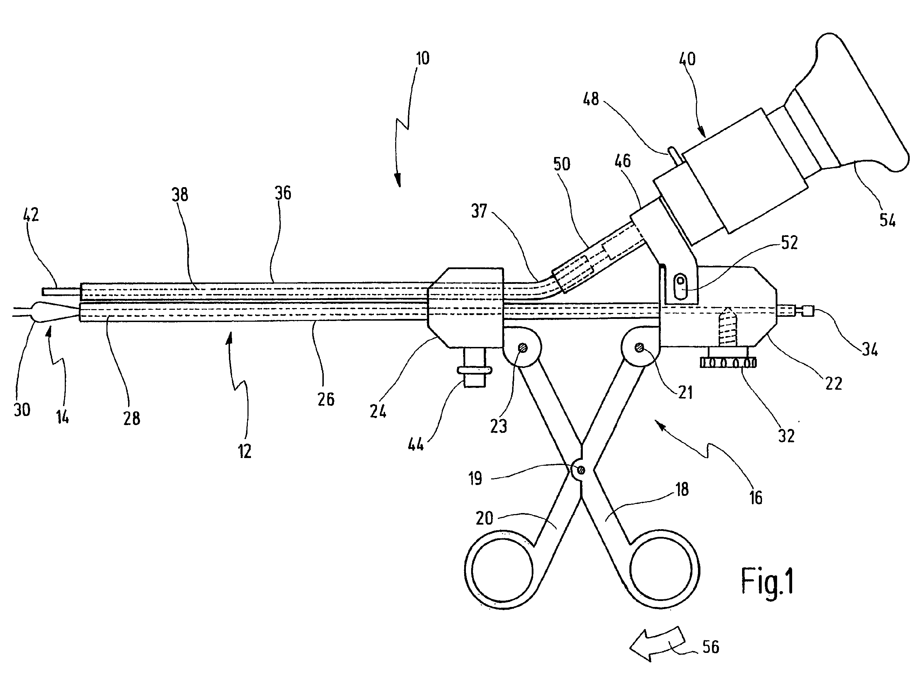

In Fig. 1 ist ein medizinisches Instrument in seiner Gesamtheit mit der Bezugsziffer 10 versehen.

Das medizinische Instrument 10 weist einen Schaft 12, an dessen distalem Ende ein Arbeitselement 14 angebracht ist, und eine Handhabe 16 auf. Die Handhabe 16 weist Scherengriffteile 18 und 20 auf, die durch eine Schraube 19 miteinander drehbar um eine Achse orthogonal zu den Scherengriffteilen 18, 20 verbunden sind. Das Scherengriffteil 18 ist über eine Schraube 21 mit einem beweglichen Teil 22 des Instruments 10 um eine Achse orthogonal zu dem Scherengriffteil 18 drehbar verbunden.

Das Scherengriffteil 20 ist durch eine Schraube 23 mit einem unbeweglichen Teil 24 des Instruments 10 um eine Achse orthogonal zu dem Scherengriffteil 20 drehbar verbunden.

Distal schließt sich an das unbewegliche Teil 24 ein Rohr 26 an, das den Schaft 12 bildet. In dem Rohr 26 verläuft ein Kraftübertragungselement 28, durch das das Arbeitselement 14 in Wirkverbindung mit dem beweglichen Teil 22 steht.

Das Kraftübertragungselement 28 dient gleichzeitig als Zuleitung einer HF-Spannung an das Arbeitselement 14, das bei diesem Instrument 10 als bipolare Zange 30 ausgebildet ist. An seinem proximalen Ende ist das Kraftübertragungselement 28 durch eine Klemmung 32 fest mit dem beweglichen Teil 22 verbunden. Die Klemmung 32 ist hierbei als Schraube ausgebildet.

Das Kraftübertragungselement 28 steht weiterhin in elektrischem Kontakt mit einer Steckverbindung 34, die sich am proximalen Ende des beweglichen Teils 22 in axialer Verlängerung des Schafts 12 befindet. Über die Steckverbindung 34 kann eine externe bipolare HF-Quelle mit dem Kraftübertragungselement 28 und damit mit der bipolaren Zange 30 verbunden werden.

Die Steckverbindung 34 ist hierbei fest mit dem Kraftübertragungselement 28, das auch als elektrischer Leiter ausgebildet ist verbunden. Die Steckverbindung 34 ist dabei kleiner als der lichte Innendurchmesser des Schafts 12. Somit kann nach Lösen der Schraube 32 das Arbeitselement 14, das Kraftübertragungselement 28 und die Steckverbindung 34 als eine Einheit durch einfaches Herausziehen am distalen Ende des Schafts 12 von dem Instrument 10 abgenommen werden.

Oberhalb des Schafts 12 befindet sich dazu parallel ein Führungsrohr 36. Das Führungsrohr 36 ersteckt sich durch das unbewegliche Teil 24 und weist proximal des unbeweglichen Teils 24 einen gekrümmten Bereich 37 auf. Innerhalb des Führungsrohrs 36 verläuft ein Endoskopschaft 38 eines Endoskops 40, das an das Instrument 10 angebracht ist, wobei ein distales Ende 42 des Endoskopschafts 38 über das Führungsrohr 36 hinausragt und sich etwa auf Höhe der bipolaren Zange 30 befindet.

Der Endoskopschaft 38 ist flexibel. Das Führungsrohr 36 dient hierbei nicht nur der Führung des Endoskopschafts 38, sondern stabilisiert diesen auch. Dadurch, dass der Endoskopschaft 38 flexibel ist, kann er sich während des Verschiebens der Form des gekrümmten Bereich 37 des Führungsrohrs 36 anpassen.

Das Führungsrohr 36 und das Rohr 26 stehen in Verbindung mit einem Spülanschluss 44, der sich an dem unbeweglichen Teil 24 befindet. Durch diesen Spülanschluss 44 kann gleichzeitig Spülflüssigkeit durch das Rohr 26 und das Führungsrohr 36 hindurchgeleitet werden.

Das Endoskop 40 ist an seinem proximalen Ende über ein Befestigungselement 46 mit dem medizinischen Instrument 10 verbunden.

Das Befestigungselement 46 weist einen Hebel 48 auf, mit dem das Befestigungselement 46 geöffnet oder geschlossen werden kann.

An seinem distalen Ende weist das Befestigungselement 46 ein Teleskoprohr 50 auf, dessen distales Ende über ein proximales Ende des Führungsrohrs 36 reicht. Im Bereich des beweglichen Teils 22 ist das Befestigungselement 46 über ein Langloch 52 mit dem beweglichen Teil 22 verbunden. Dadurch ist das Befestigungselement 46 quer zum Schaft 12 geringfügig beweglich.

Das Befestigen des Endoskops 40 erfolgt durch Einführen des Endoskops 40 durch das Befestigungselement 46, wobei der Endoskopschaft 38 in das Führungsrohr 36 eingeführt wird, und durch Umlegen des Hebels 48, der einen Bajonettverschluss schließt.

Ein Okular 54 des Endoskops 40 befindet sich außerhalb der Längsachse des Schafts 12, in deren Verlängerung sich die Steckverbindung 34 befindet.

Wird nun das Scherengriffteil 18 in Richtung des Pfeils 56 bewegt, wird das bewegliche Teil 22 gegenüber dem unbeweglichen Teil 24 nach proximal verschoben. Dadurch wird das Arbeitselement 14 ebenfalls Richtung proximal, d.h. in Richtung des Rohrs 26 verschoben. Die bipolare Zange 30 wird dabei in das Rohr 26 verschoben, wodurch Maulteile der Zange aufeinander zu bewegt werden, wodurch die bipolare Zange 30 geschlossen wird.

Das Endoskop 40 wird ebenfalls Richtung proximal verschoben, wodurch sich das distale Ende 42 des Endoskopschafts 38 in Richtung des Führungsrohrs 36 verschiebt. Dadurch, dass das Teleskoprohr 50 entlang des gekrümmten Bereichs 37 bewegt wird, wird das proximale Ende des Endoskops 40 und damit verbunden das Befestigungselement 46 entlang des Langlochs 52 geringfügig angehoben.

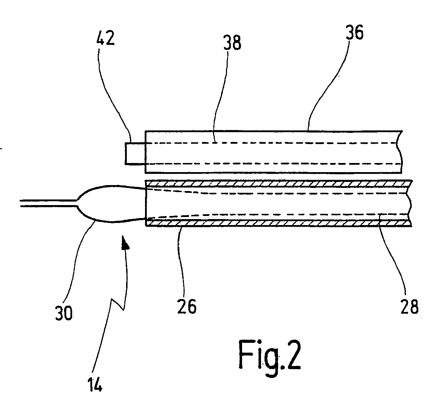

Fig. 2 zeigt eine Vergrößerung des distalen Abschnitts des medizinischen Instruments 10, nachdem das Scherengriffteil 18 in Richtung des Pfeils 56 bewegt wurde. Das Arbeitselement 14 wurde in das Rohr 26 hinein verschoben, wodurch die Maulteile der bipolaren Zange 30 zusammengedrückt werden. Gleichzeitig wurde das distale Ende 42 des Endoskopschafts 38 proximal in Richtung des Führungsrohrs 36 verschoben. Bei diesem Verschieben ist der Abstand zwischen dem distalen Ende 42 des Endoskopschafts 38 und der bipolaren Zange 30 gleich geblieben.

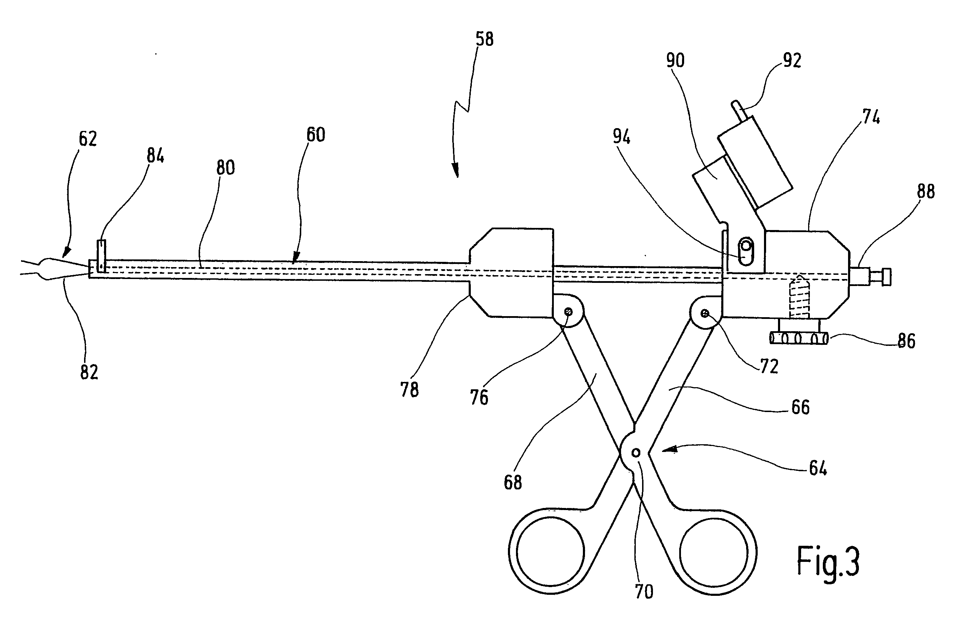

In Fig. 3 ist ein weiteres Ausführungsbeispiel eines medizinischen Instruments in seiner Gesamtheit mit der Bezugsziffer 58 versehen.

Das medizinische Instrument 58 weist einen Schaft 60, an dessen distalem Ende ein Arbeitselement 62 angebracht ist, und eine Handhabe 64 auf. Die Handhabe 64 besteht aus Scherengriffteilen 66 und 68, die über eine Schraube 70 orthogonal zur Ebene der Scherengriffteile 66 und 68 um eine Achse drehbar verbunden sind.

Das Scherengriffteil 66 ist durch eine Schraube 72 mit einem beweglichen Teil 74 des Instruments 58 drehbar verbunden. Das Scherengriffteil 68 ist durch eine Schraube 76 mit einem unbeweglichen Teil 78 des Instruments 58 verbunden.

Durch den Schaft 60 verläuft ein Kraftübertragungselement 80, das an seinem distalen Ende mit dem Arbeitselement 62 in Verbindung steht, das in diesem Fall als bipolare Zange 82 ausgebildet ist. Das Kraftübertragungselement 80 dient gleichzeitig als Zuleitung für eine HF-Spannung zu der bipolaren Zange 82.

Ebenfalls am distalen Ende des Schafts 60 angebracht ist ein Befestigungselement 84 zum Aufnehmen eines Schafts eines Endoskops.

An seinem proximalen Ende ist das Kraftübertragungselement 80 durch eine Klemmung 86 kraftschlüssig mit dem beweglichen Teil 74 verbunden. Die Klemmung 86 ist hierbei als Schraube ausgebildet. Die bipolare Zange 82 und damit verbunden das Kraftübertragungselement 80 können nach Lösen der Schraube einfach am distalen Ende aus dem Schaft 60 des medizinischen Instruments 58 entnommen werden.

Das Kraftübertragungselement 80 steht außerdem in elektrischem Kontakt mit einer Steckverbindung 88, die proximal in axialer Verlängerung des Schafts 60 an dem beweglichen Teil 74 angebracht ist.

Weiterhin ist an dem beweglichen Teil 74 ein Befestigungselement 90 angebracht.

Dieses Befestigungselement 90 weist einen Hebel 92 auf, mit dem ein Endoskop kraftschlüssig mit dem Befestigungselement 90 verbunden werden kann.

Im Bereich der Verbindung mit dem beweglichen Teil 74 weist das Befestigungselement 90 ein Langloch 94 auf. Dadurch ist das Befestigungselement 90 orthogonal zum Schaft 60 gegenüber dem beweglichen Teil 74 verschiebbar.

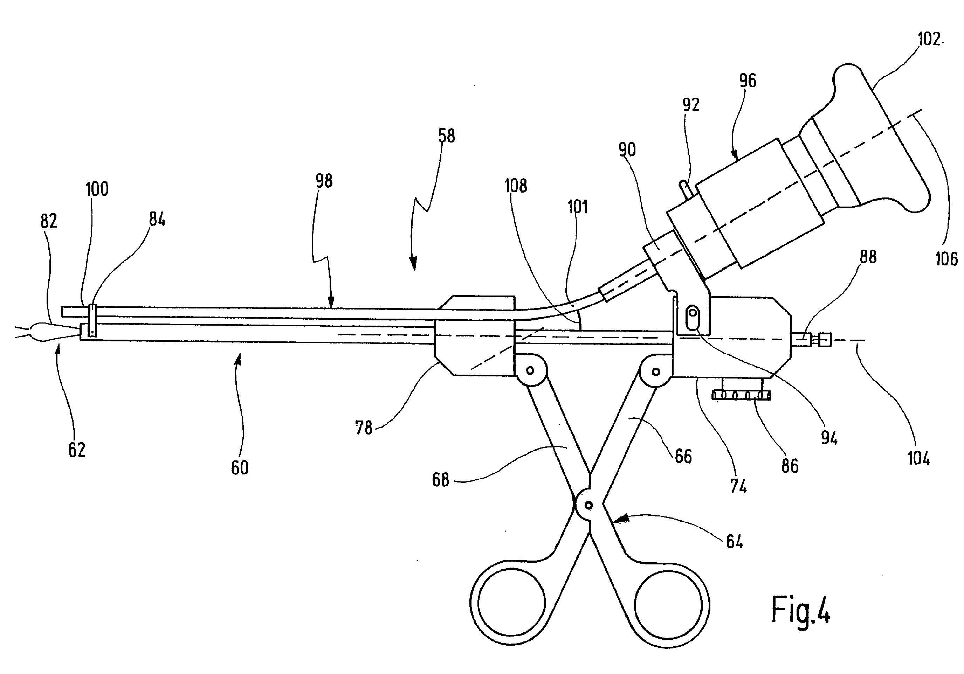

In Fig. 4 ist ein medizinisches Instrument 58 dargestellt, an das ein Endoskop 96 angebracht wurde.

Ein Endoskopschaft 98 des Endoskops 96 verläuft hierbei im Wesentlichen parallel zum Schaft 60 des Instruments 58. An einem distalen Ende 100 des Endoskopschafts 98 ist dieser durch das Befestigungselement 84 mit dem distalen Ende des Schafts 60 des medizinischen Instruments 58 verbunden.

Das distale Ende 100 des Endoskopschafts 98 steht hierbei über das distale Ende des Schafts 60 des Instruments 58 hinaus und befindet sich in etwa auf Höhe der bipolaren Zange 82. Im proximalen Bereich weist der Endoskopschaft 98 einen gekrümmten Bereich 101 auf. Durch diesen gekrümmten Bereich 101 liegt ein Okular 102 des Endoskops 96 außerhalb einer Längsachse 104 des Schafts 60 des Instruments 58, die hier durch eine gestrichelte Linie dargestellt ist.

Der Endoskopschaft 98 ist im Bereich des gekrümmten Bereichs 101 flexibel. Ein Abschnitt des Endoskopschafts 98 distal des gekrümmten Bereichs 101 ist starr. Somit kann eine Auslenkung des Endoskopschafts 98 in dem gekrümmten Bereich 101 verändert werden, wobei der starre Abschnitt ohne zusätzliche Führung parallel zum Schaft 60 des Endoskops 58 angebracht werden kann.

Die Längsachse 104 des Schafts 60 bildet zusammen mit einer Sichtlinie 106 des Okulars 102, die hier als gestrichelte Linie dargestellt ist, einen Winkel 108.

Da der Endoskopschaft 98 in den gekrümmten Bereich 101 flexibel ist, kann der Winkel 108 durch Verschieben des Befestigungselements 90 entlang des Langlochs 94 verändert und durch hier nicht dargestellte Mittel fixiert werden.

## Patentansprüche

1. Medizinisches Instrument, mit einem Schaft (12; 60), einem Arbeitselement (14; 62) am distalen Ende des Schaftes (12; 60), einer Handhabe (16; 64) mit zumindest einem beweglichen Teil (22; 74) zum Betätigen des Arbeitselements (14; 62) und mit einem Befestigungselement (46; 90) zum Befestigen eines Endoskops (40; 96), das einen Endoskopschaft (38; 98) und ein Okular (54; 102) aufweist, wobei das distale Ende (42; 100) des Endoskops (40; 96) im Bereich des distalen Endes des Schaftes (12; 60) und das proximale Ende des Endoskops (40; 96) im Bereich der Handhabe (16; 64) des Instruments (10; 58) anbringbar sind und das Endoskop (40; 96) in Längsrichtung des Schaftes (12; 60) verschiebbar ist, **dadurch gekennzeichnet, dass** das Befestigungselement (46; 90) so ausgebildet ist, dass das Endoskop (40; 96) an der Außenseite des Instruments (10; 58) anbringbar ist, wobei das Okular (54; 102) des Endoskops (40; 96) im am Instrument angebrachten Zustand außerhalb der Längsachse (104) des Schaftes (12; 60) des Instruments (10; 58) liegt.

2. Medizinisches Instrument nach Anspruch 1, **dadurch gekennzeichnet, dass** das Arbeitselement (14; 62) zumindest eine mit einer HF-Spannung beaufschlagbare Elektrode aufweist.

3. Medizinisches Instrument nach Anspruch 2, **dadurch gekennzeichnet, dass** das Arbeitselement (14; 62) als bipolares Arbeitselement (14; 62) ausgebildet ist.

4. Medizinisches Instrument nach einem der Ansprüche 1 bis 3, **dadurch gekennzeichnet, dass** das Arbeitselement (14; 62) als bipolare Zange (30; 82) ausgebildet ist.

5. Medizinisches Instrument nach einem der Ansprüche 2 bis 4, **dadurch gekennzeichnet, dass** das Arbeitselement (14; 62) mit einem elektrischen Kontakt am proximalen Ende des Instruments (10; 58) in Verbindung steht, wobei der elektrische Kontakt auf der Längsachse (104) des Schafts (12; 60) liegt.

6. Medizinisches Instrument nach Anspruch 5, **dadurch gekennzeichnet, dass** das Arbeitselement (14; 62) durch zumindest einen Leiter mit dem elektrischen Kontakt verbunden ist, wobei das Arbeitselement (14; 62), der zumindest eine Leiter und der elektrische Kontakt als eine Einheit aus dem Schaft (12; 60) entnehmbar sind.

7. Medizinische Instrument nach Anspruch 5 oder 6, **dadurch gekennzeichnet, dass** der elektrische Kontakt als Steckverbindung (34; 88) zum Anschluss eines externen Kabels ausgebildet ist, wobei die Steckverbindung (34; 88) im Wesentlichen in geradliniger Verlängerung des Schafts (12; 60) angebracht ist.

8. Medizinisches Instrument nach einem der Ansprüche 1 bis 7, **dadurch gekennzeichnet, dass** am Schaft (12) ein Führungsrohr (36) für den Endoskopschaft (38) angeordnet ist, das sich parallel zum Schaft (12; 60) erstreckt und in das der Endoskopschaft (38; 98) einführbar ist.

9. Medizinisches Instrument nach Anspruch 8, **dadurch gekennzeichnet, dass** das Führungsrohr (36) durch einen Kanal im Schaft (12; 60) gebildet wird.

10. Medizinisches Instrument nach Anspruch 8 oder 9, **dadurch gekennzeichnet, dass** es einen Spülanschluss (44) aufweist, über den eine Innenseite des Schafts (12; 60) und des Führungsrohrs (36) gleichzeitig gespült werden kann.

11. Medizinisches Instrument nach einem der Ansprüche 1 bis 10, **dadurch gekennzeichnet, dass** das Befestigungselement (46; 90) mit dem beweglichen Teil (22; 74) der Handhabe (16; 64) in Wirkverbindung steht.

12. Medizinisches Instrument nach einem der Ansprüche 1 bis 11, **dadurch gekennzeichnet, dass** das Befestigungselement (46; 90) bei Betätigen der Handhabe (16; 64) das distale Ende (42; 100) des Endoskops (40; 96) in konstantem Abstand zum distalen Ende des Arbeitselements (14; 62) hält.

13. Medizinisches Instrument nach einem der Ansprüche 1 bis 12, **dadurch gekennzeichnet, dass** die Handhabe (16; 64) zumindest einen Griff aufweist, der seitlich von dem Instrument (10; 58) absteht.

14. Medizinisches Instrument nach Anspruch 13, **dadurch gekennzeichnet, dass** der Griff der Handhabe (16; 64) in der Art von Scherengriffteilen (18, 20; 66, 68) ausgebildet ist.

15. Medizinisches Instrument nach einem der Ansprüche 1 bis 14, **dadurch gekennzeichnet, dass** ein Schaft des Endoskops in einem proximalen Abschnitt eine Krümmung aufweist und zumindest in dem gekrümmten Bereich flexibel oder biegsam ist.

## Claims

1. A medical instrument, comprising a shaft (12; 60), a work element (14; 62) at the distal end of the shaft (12; 60), a handle (16; 64) with at least one movable part (22; 74) for actuating the work element (14; 62), and a securing element (46; 90) for securing an endoscope (40; 96) which has an endoscope shaft (38; 98) and an eyepiece (54; 102), the distal end (42; 100) of the endoscope (40; 96) being able to be arranged in the area of the distal end of the shaft (12; 60), and the proximal end of the endoscope (40; 96) being able to be arranged in the area of the handle (16; 64) of the instrument (10; 58), and the endoscope (40; 96) being displaceable in the longitudinal direction of the shaft (12; 60), **characterized in that** the securing element (46; 90) is designed in such a way that the endoscope (40; 96) can be arranged on the outside of the instrument (10; 58), the eyepiece (54; 102) of the endoscope (40; 96), in the state when the latter is arranged on the instrument, lying outside the longitudinal axis (104) of the shaft (12; 60) of the instrument (10; 58).

2. The medical instrument of claim 1, **characterized in that** the work element (14; 62) comprises at least one electrode that can be acted upon with HF voltage.

3. The medical instrument of claim 2, **characterized in that** the work element (14; 62) is designed as a bipolar work element (14; 62).

4. The medical instrument of anyone of claims 1 through 3, **characterized in that** the work element (14; 62) is designed as a bipolar forceps (30; 82).

5. The medical instrument of anyone of claims 2 through 4, **characterized in that** the work element (14; 62) communicates with an electrical contact at the proximal end of the instrument (10; 58), the electrical contact lying on the longitudinal axis (104) of the shaft (12; 60).

6. The medical instrument of claim 5, **characterized in that** the work element (14; 62) is connected to the electrical contact via at least one conductor, it being possible for the work element (14; 62), the at least one conductor and the electrical contact to be removed as one unit from the shaft (12; 60).

7. The medical instrument of claim 5 or 6, **characterized in that** the electrical contact is designed as a plug connection (34; 88) for attachment of an external cable, the plug connection (34; 88) being arranged in a substantially rectilinear continuation of the shaft (12; 60).

8. The medical instrument of anyone of claims 1 through 7, **characterized in that** a guide tube (36) for the endoscope shaft (38) is arranged on the shaft (12) and extends parallel to the shaft (12; 60), and the endoscope shaft (38; 98) can be inserted into it.

9. The medical instrument of claim 8, **characterized in that** the guide tube (36) is formed by a channel in the shaft (12; 60).

10. The medical instrument of claim 8 or 9, **characterized in that** it comprises an irrigation attachment piece (44) via which an inside of the shaft (12; 60) and of the guide tube (36) can be simultaneously irrigated.

11. The medical instrument of anyone of claims 1 through 10, **characterized in that** the securing element (46; 90) is in operative connection with the movable part (22; 74) of the handle (16; 64).

12. The medical instrument of anyone of claims 1 through 11, **characterized in that**, upon actuation of the handle (16; 64), the securing element (46; 90) holds the distal end (42; 100) of the endoscope (40; 96) at a constant distance from the distal end of the work element (14; 62).

13. The medical instrument of anyone of claims 1 through 12, **characterized in that** the handle (16; 64) comprises at least one grip protruding laterally from the instrument (10; 58).

14. The medical instrument of claim 13, **characterized in that** the grip of the handle (16; 64) is designed in the manner of scissor grip parts (18, 20; 66, 68).

15. The medical instrument of anyone of claims 1 through 14, **characterized in that** a shaft of the endoscope has a curvature in a proximal portion and is flexible or bendable at least in the curved area.

## Revendications

1. Instrument médical, comportant une tige (12 ; 60), un élément de travail (14; 62) sur l'extrémité distale de la tige (12 ; 60), une manette (16; 64) avec au moins une partie mobile (22 ; 74) pour manoeuvrer l'élément de travail (14 ; 62), et un élément de fixation (46 ; 90) pour la fixation d'un endoscope (40 ; 96), qui comporte une tige d'endoscope (38 ; 98) et un oculaire (54 ; 102), l'extrémité distale (42 ; 100) de l'endoscope (40; 96) étant destinée à être montée dans la zone de l'extrémité distale de la tige (12 ; 60), et l'extrémité proximale de l'endoscope (40 ; 96) étant destinée à être montée dans la zone de la manette (16 ; 64) de l'instrument (10 ; 58), et l'endoscope (40 ; 96) étant mobile dans le sens longitudinal de la tige (12 ; 60), **caractérisé en ce que** l'élément de fixation (46 ; 90) est conçu de telle sorte que l'endoscope (40 ; 96) peut être monté sur le côté extérieur de l'instrument (10 ; 58), l'oculaire (54 ; 102) de l'endoscope (40 ; 96), à l'état monté sur l'instrument, étant situé à l'extérieur de l'axe longitudinal (104) de la tige (12 ; 60) de l'instrument (10 ; 58).

2. Instrument médical selon la revendication 1, **caractérisé en ce que** l'élément de travail (14 ; 62) comporte au moins une électrode apte à être sollicitée par une tension à haute fréquence.

3. Instrument médical selon la revendication 2, **caractérisé en ce que** l'élément de travail (14 ; 62) est réalisé sous forme d'élément de travail (14; 62) bipolaire.

4. Instrument médical selon l'une quelconque des revendications 1 à 3, **caractérisé en ce que** l'élément de travail (14 ; 62) est réalisé sous forme de pince (30 ; 82) bipolaire.

5. Instrument médical selon l'une quelconque des revendications 2 à 4, **caractérisé en ce que** l'élément de travail (14 ; 62) est en liaison avec un contact électrique au niveau de l'extrémité proximale de l'instrument (10 ; 58), ledit contact électrique étant situé sur l'axe longitudinal (104) de la tige (12 ; 60).

6. Instrument médical selon la revendication 5, **caractérisé en ce que** l'élément de travail (14 ; 62) est relié au contact électrique via au moins un conducteur, l'élément de travail (14 ; 62), ledit au moins un conducteur et le contact électrique pouvant être retirés sous la forme d'une unité hors de la tige (12 ; 60).

7. Instrument médical selon la revendication 5 ou 6, **caractérisé en ce que** le contact électrique est réalisé sous la forme d'une fiche de connexion (34 ; 88) pour le branchement d'un câble externe, la fiche de connexion (34 ; 88) étant montée sensiblement dans le prolongement rectiligne de la tige (12 ; 60).

8. Instrument médical selon l'une quelconque des revendications 1 à 7, **caractérisé en ce que** sur la tige (12) est agencé un tube de guidage (36) pour la tige d'endoscope (38), lequel est orienté parallèlement à la tige (12 ; 60) et dans lequel la tige d'endoscope (38 ; 98) peut être introduite.

9. Instrument médical selon la revendication 8, **caractérisé en ce que** le tube de guidage (36) est formé par un conduit dans la tige (12 ; 60).

10. Instrument médical selon la revendication 8 ou 9, **caractérisé en ce qu'**il comporte un raccord de lavage (44), par l'intermédiaire duquel une face intérieure de la tige (12 ; 60) et une face intérieure du tube de guidage (36) peuvent être lavées simultanément.

11. Instrument médical selon l'une quelconque des revendications 1 à 10, **caractérisé en ce que** l'élément de fixation (46 ; 90) est en liaison active avec la partie mobile (22 ; 74) de la manette (16 ; 64).

12. Instrument médical selon l'une quelconque des revendications 1 à 11, **caractérisé en ce que** l'élément de fixation (46 ; 90), lors de la manipulation de la manette (16 ; 64), maintient l'extrémité distale (42, 100) de l'endoscope (40 ; 96) à une distance constante de l'extrémité distale de l'élément de travail (14; 62).

13. Instrument médical selon l'une quelconque des revendications 1 à 12, **caractérisé en ce que** la manette (16 ; 64) comporte au moins une poignée, qui est en saillie sur le côté de l'instrument (10 ; 58).

14. Instrument médical selon la revendication 13, **caractérisé en ce que** la poignée de la manette (16 ; 64) est réalisée sous la forme de branches de ciseaux (18, 20 ; 66, 68).

15. Instrument médical selon l'une quelconque des revendications 1 à 14, **caractérisé en ce qu'**une tige de l'endoscope comporte, dans une partie proximale, une courbure et au moins une zone courbe flexible ou pliable.
